# EUROPEAN PATENT SPECIFICATION

(11) **EP 3 478 229 B1**
(45) Date of publication and mention of the grant of the patent: **25.11.2026**
(21) Application number: 17823759.0
(22) Date of filing: 02.07.2017
(51) Int. Cl.: A61F 5/56, A61F 5/37, A61F 5/055, A61G 1/04, A61G 7/07, A61G 13/12

(54) **DEVICE FOR OPTIMAL AIRWAY OPENING**
VORRICHTUNG ZUR OPTIMALEN ATEMWEGSÖFFNUNG
DISPOSITIF D'OUVERTURE OPTIMALE DES VOIES RESPIRATOIRES.

(30) Priority: 03.07.2016 US 201662358006 P
(43) Date of publication of application: 08.05.2019
(73) Proprietor: Inovytec Medical Solutions Ltd., 4501306 Hod Hasharon (IL)
(72) Inventor: KANTOR, Ehud, 4531409 Hod Hasharon (IL)
(74) Representative: Schulz Junghans Patentanwälte PartGmbB
(86) International application number: PCT/IL2017/050738
(87) International publication number: WO 2018/008016

(56) References cited:
- WO-A1-2015/025319
- CA-A1- 2 916 907
- GB-A- 1 290 523
- JP-A- 2010 148 927
- JP-B2- 5 615 849
- US-A- 5 524 639
- US-A1- 2011 036 355
- US-A1- 2011 253 150
- US-A1- 2016 101 008
- US-A1- 2016 101 008
- US-A1- 2016 151 222
- US-B1- 7 055 524
- US-B2- 7 350 250

## Description

### Field of the invention

The invention is from the field of medical devices. Specifically the invention relates to devices for opening an airway in a patient.

### Background of the Invention

Airway obstruction is a common and potentially devastating complication in certain medical conditions such as post-surgery, apnea, trauma, airway obstruction and more.

The most frequent cause of airway obstruction is the loss of pharyngeal tone in an unconscious, sedated or obtunded patient. When the muscles of the tongue and mouth floor relax, the tongue lies close to or on the back wall of the oropharynx. The epiglottis may obstruct the glottic opening or seal against the back wall of the pharynx, as well.

All invasive methods to maintain an open airway involve devices that are inserted into the airway and mechanically supply an open tube aimed to maintain an open airway. There are several non-invasive positioning maneuvers known in the art which can be performed to relieve the soft tissue obstruction and improve air flow. One non-invasive mean to protect the airway in an airway obstructed patient is by pushing the jaw forward in what is known in the art as a "Jaw thrust maneuver" in which the mandibles are pushed forward to prevent backward collapse of the mandible and suffocation.

Another important factor for obtaining an open airway is the angle of the patient's neck. It is important to prevent the head of the treated individual from assuming unwanted positions which may not fully support an open airway or may even obstruct the airway as often happens when a pillow is placed behind the head. In order to have optimal opening of the airway there is a need to tilt the head of the subject backwards in a suitable angle. Tilting of the head results in neck extension which opens any obstruction caused by relaxation of the soft tissues of the oropharynx and thus ensures an open airway.

Optimal airway opening is obtained through a delicate balance between the two positioning maneuvers described above. Therefore, there is an unmet need for a device which is capable of simultaneously performing both actions of head tilt and jaw thrust maneuver.

During this action in order to better control the airway opening on a syncope or pre-syncope patient in a supine position it is advantageous to fixate the patient's head. The fixation of the head should be done when the optimal airway opening is achieved. The importance of maintaining the head in a fixed position is in order to obtain control of the spine after orthopedic surgery or trauma, to allow optimal intubation in a pre and during anesthetic surgery and to maintain the optimal position for the airway opening.

It is therefore a purpose of the present invention to provide a device which is capable of simultaneously performing both actions of head tilt and jaw thrust maneuver.

It is another purpose of the invention to provide an airway support device for post anesthesia surgeries, recovery rooms, and sedation procedures overcoming the common airway obstruction and potentially devastating complications in the post operative period.

Further purposes and advantages of this invention will appear as the description proceeds.

US 2016 101008 A1 discloses a similar device for opening an maintaining an open airway in a subject and is considered the prior art closest to the subject-matter of claim 1, disclosing a pair of pads held against the remus of a patient's jaw, to prevent the jaw from slipping back and causing an airway obstruction, while the patient's neck is hyperextended to also cause the patient's airway to stay open. The device including the adjustable jaw pads as well as a triangularly shaped portion over which the patient's neck rests is not required to be attached to the surface on which the patient is lying, and permits the patient to be rolled on either side while still maintaining the patency of the patient's airway.

### Summary of the Invention

The invention is a wearable device according to claim 1. Advantageous embodiments are defined in the dependent claims.

All the above and other characteristics and advantages of the invention will be further understood through the following illustrative and non-limitative description of embodiments thereof, with reference to the appended drawings.

### Brief Description of the Drawings

- Fig. 1A schematically shows an example of the device;
- Fig. 1B schematically shows the direction of the forces exerted on the head of a subject by the device of Fig. 1A when the device is activated to simultaneously carry out head tilt and jaw thrust maneuvers;
- Fig.2 is a schematic view from the left side of the head 14 of the subject with the device of Fig. 1A attached;
- Fig. 3 is a schematic view from the right side of the head 14 of the subject with the device of Fig. 1A attached;
- Fig. 4 is a schematic view from the back of the head 14 of the subject with the device of Fig. 1A attached;
- Fig. 5A and Fig. 5B schematically show a device of Fig. 1A attached to a horizontal surface.

### Detailed Description of Embodiments of the Invention

The present invention is a wearable device for opening an airway in a patient. More specifically the device of the present invention combines a mechanism which is configured for modifying a head angle of the subject when in the supine position in order to achieve a head tilt position (also known herein as a head tilt mechanism), and a mechanism for causing a forward movement of the mandibles (also known herein as a jaw thrust mechanism) in order to obtain an optimal open airway. Head tilt and jaw thrust can be performed either separately or simultaneously.

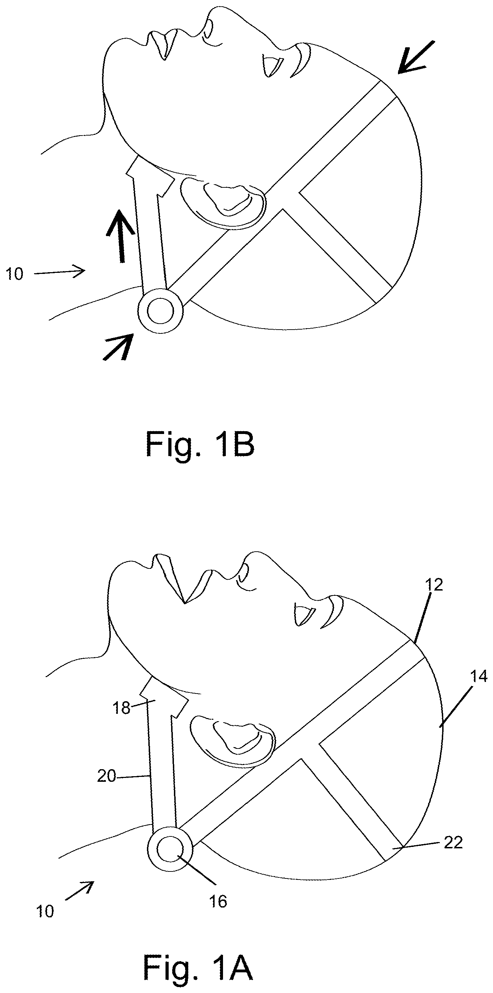

The device is schematically shown in Fig. 1A. Device 10 comprises a flexible primary strap 12 configured to be worn on the head 14 of a subject passing over the forehead and the neck as shown in Fig. 1A. One side of primary strap 12 is connected to a head tilt mechanism, which will be described in detail herein below. The head tilt mechanism is attached to a semi-rigid element which is positioned behind the neck and is configured to support the neck (hereinafter "neck support" 16). The device 10 may include a secondary strap 22 connected to the primary strap 12 and running over the top of the head 14 to improve the attachment of the device 10 to the head 14.

Mandible engaging elements 18 configured to match the shape of the mandibles are pivotally connected on both sides of the head to components of a jaw thrust mechanisms 20, which are affixed to the neck support 16. In embodiments of the device each of the mandible engagement elements 18 can be fixed to the mandible by stickers or vacuum. The jaw thrust mechanisms 20 are configured to move the mandible engaging elements 18 forward in order to perform a jaw thrust maneuver. Embodiments of the neck support 16, the head tilt mechanism and the jaw thrust mechanism will be described in detail herein below.

The arrows in Fig. 1B schematically show respectively the directions of the forces exerted by primary strap 12, neck support 16, and mandible engaging elements 18 on the head 14 and lower jaw of the patient when device 10 of Fig. 1A is activated to simultaneously carry out head tilt and jaw thrust maneuvers.

In another example of device 10 the primary strap 12 passes over the nose bridge and the neck of the subject instead of over the forehead as shown in Figs. 1A and 1B.

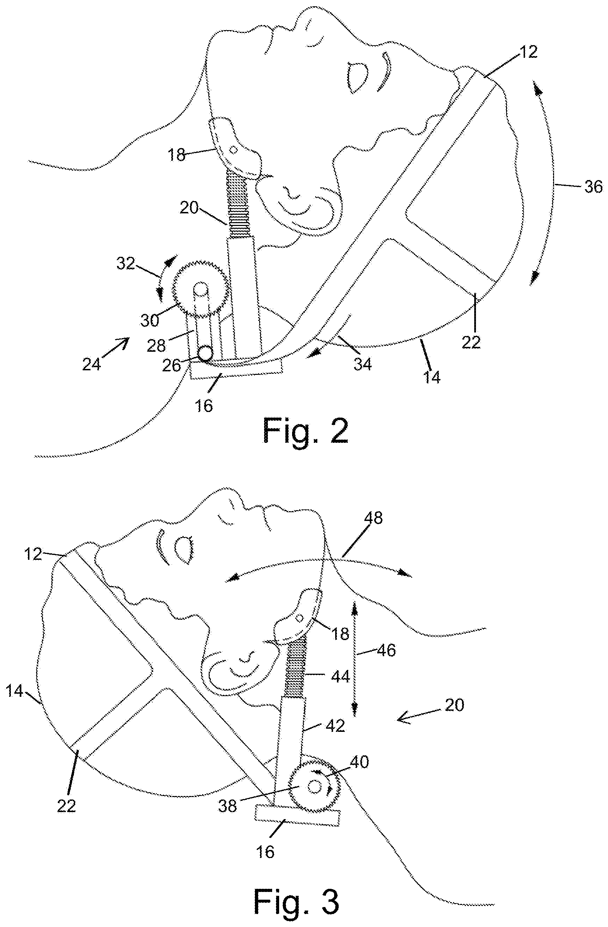

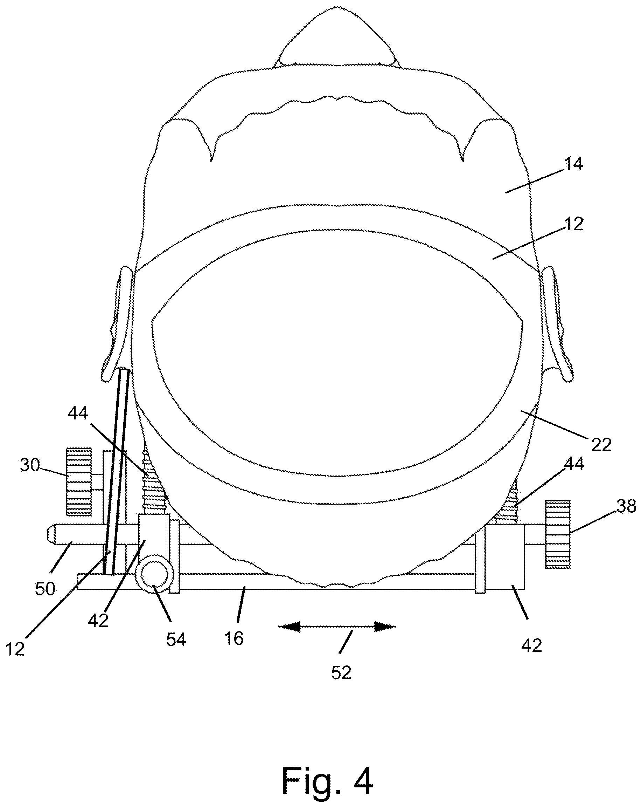

Fig.2 is a schematic view from the left side, Fig. 3 is a schematic view from the right side, and Fig. 4 is a schematic view from the back showing the device of Fig. 1A attached to the head 14 of a subject.

Fig. 2 illustrates how the head tilt mechanism operates to cause head 14 to tilt up and down in the direction shown by arrow 36. The end of primary strap 12 on the right side of head 14 is firmly affixed to neck support 16. The end of primary strap 12 on the left side of head 14 is firmly affixed to pulley 26. An axel of pulley 26 is connected by belt 28 to the axle of rotatable handle 32, which can be rotated clockwise or counterclockwise as shown by arrow 32. Rotating handle 32 in the clockwise direction pulls the end of primary strap 12 in the direction shown by arrow 34 and wrapping the end of around of primary strap 12 around pulley 26 effectively applying a force via primary strap 12 that pulls head 14 downwards. Rotating handle 32 counterclockwise unwraps primary strap 12 from around pulley 26 effectively reducing the force that primary strap 12 exerts on the head allowing the head to move upwards. The head tilt mechanism comprises a locking mechanism, e.g. a pin or pawl, which maintains the head at an optimal angle. In other embodiments a gear train can replace belt 28 that transfers force from handle 30 to pulley 26. Handle 30 can be rotated either manually or by a motor, e.g. an electric stepping motor.

Fig. 3 and Fig. 4 illustrate the jaw thrust mechanism 20, which, in the example shown comprises two drive mechanisms 42 that are connected by a shaft 50 having a handle 38 affixed to one side and gears (not shown inside a housing of the drive mechanisms) that mate with the drive mechanism 42. Rotating handle 38 clockwise or counterclockwise advances or withdraws elongated members 44, which in the embodiment shown are screws in the direction indicated by arrow 46 pushing the mandible engaging elements 18 upwards or lowering them to move the jaw in the direction indicated by arrow 48. In order to be used with subjects having different dimensions, the distance between drive mechanisms 42 can be adjusted as indicated by arrow 52. The button 54 symbolically represents a locking mechanism configured to lock the jaw thrust mechanism 20 to maintain optimal jaw thrust. Other examples of the elongated members 44 can comprise other types of mechanical arrangement for raising and lowering mandible engaging elements 18, e.g. a rack and pinion gear. Handle 40 can be rotated either manually or by a motor, e.g. an electric stepping motor.

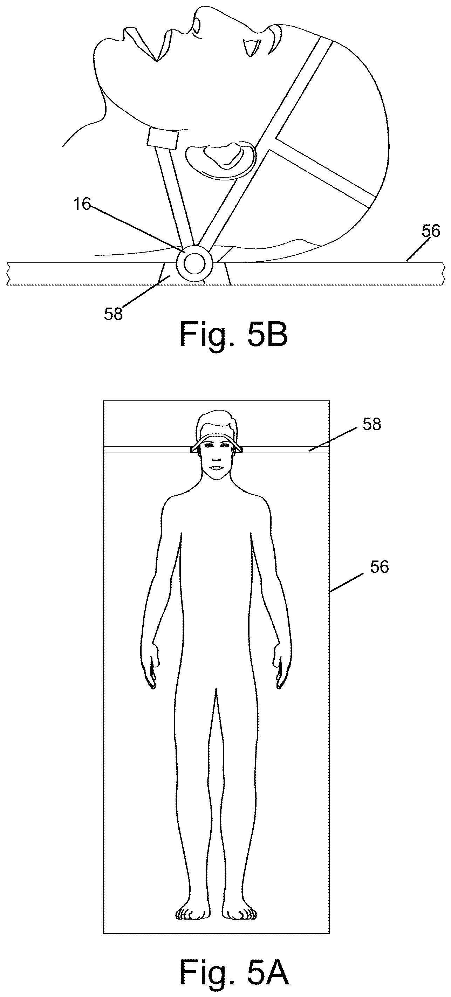

The device may be configured to be attached to a bed a stretcher or any other surface 56 in order to improve the fixation of the patients head as shown in Fig 5A. The fixation of the head is beneficial in maintaining the required neck angle for optimal opening of the airway which is achieved both by the jaw thrust maneuver and the head tilt. In order to achieve this, the device may include connectors that allow attachment of the device to the surface. In an embodiment the connectors are attached to the device through connection to the neck support portion of the device at each extremity and are configured to be connected to two anchorage points, which are positioned on the surface at both sides of the patient's neck. The distance between the two anchorage points on the surface 56 may be adjustable to the size of a patient's neck by allowing their movement on the horizontal axis of the surface 56. This may be achieved by forming the anchorage points on two elements that are configured to be attached to a rail on the surface, e.g. a dovetail rail 58 as shown in Fig. 5B.

the anchorage points may be an integral part of the surface such as in the case of a hospital bed or a stretcher. Alternatively, a universal mounting device is configured to be firmly attached to a flat surface of choice. The universal mounting device comprises two adjustable anchorage points as described above. After attachment of the mounting device to the desired surface the gap between the two anchorage points may be adjusted to the size of a subject's neck. Once adjusted the connectors on the neck support of the head mounted device are connected to the anchorage points on the mounting device in order to achieve fixation of the patient's head to any chosen surface. The device 10 may also include a head support, which is mounted on the surface and supports the distal part of the patient's head. The head support may be a cushion or a rigid element that is connected to the surface either directly or as part of the universal mounting device that is configured to be attached to a flat surface of choice.

As said, the operation of the airway opening mechanism is either manual or powered by motors. Opening of the airway is done in response to measurements of the patient's condition by sensors such as pulse oximeter, audio sensors or any other sensor configured to evaluate the state of respiration of the patient and to react to a state of respiratory emergency by automatically opening an airway through optimization of jaw thrust maneuver and/or head tilt.

The intention of the inventors is for the device to be used by an anesthetist to aid in intubation while treating a patient before a surgical procedure, to continue to accompany the patient during the operation and after the operation to preserve an open airway during recovery in the recovery room.

The anesthetist is able to make use of the device to aid in the process of intubation. The device will be implemented on the patient and secured to the operating table as described herein above. The device, when fastened to the table, will allow the anesthetist, by rotating the two handles (30,38) to move the jaw forward (JAW THRUST) and to tilt the head just the amounts needed to position the epiglottis ideally to carry out intubation and to introduce the tube into the trachea.

After the operation and removal of the anesthesia tube, the device will maintain the airway of the patient open during the recovery process. After the patient wakes up, the device can be unsecured from the operating table but will remain on him/her until the device is no longer needed to maintain an open airway.

For Ear-Nose-Throat procedures the device can be used not only to aid in intubation but also to prevent movement of the head and the mouth open according to the medical requirements.

The device can also be used to aid in maintaining open upper airways during operations carried out with partial anesthesia, such as in cosmetic surgery. For operations carried out with local anesthesia in which intubation is not required, the device can be used wither fastened or not fastened to the bed. By rotating the handles (30,38) the epiglottis can be positioned to maintain the upper airways open.

Additionally the device can also be used to aid in maintaining open upper airways for patients undergoing examinations, such as MRI and CT and for patients in hospital wards that suffer from breathing problems, such a shortness of breath, apnea, and lung diseases.

## Claims

1. A wearable device (10) for opening and maintaining an open airway in a subject, the device (10) comprising:
a) a semi-rigid neck support (16) that is configured to be positioned behind the neck and is configured to support the neck of the subject;
b) a head tilt mechanism (24) located on one side of the subject affixed to the neck support (16) comprising a rotatable handle (30) to perform a head tilt maneuver by modifying a head angle of the subject when in the supine position in order to achieve a head tilt position;
c) mandible engaging element (18) configured to match the shape of the mandibles;
d) a jaw thrust mechanism (20) attached to the neck support (16) comprising two drive mechanisms (42) that are connected by a shaft (50) having a rotatable handle (38) affixed to one end of the shaft (50) and gears that mate with the drive mechanism (42), two elongated members (44), one projecting upwards from each drive mechanism (42) and configured to be raised or lowered in relation to the neck support (16), the mandible engaging elements (18) pivotally attached to the upper end of a different one of each of the elongated members (44);
e) a flexible primary strap (12) connected to the rotatable handle (30) having a first end affixed to the pulley (26) and a second end affixed to the nek support (16) opposite the side at which the head tilt mechanism (24) is affixed to the neck support (16), the flexible primary strap (12) is configured to wrap around the pulley (26) to pull the head (14) downwards when the rotatable handle (30) is rotated in a first direction or to allow the head (14) to move upwards when the rotatable handle (30) is rotated in an opposite direction;
f) sensors configured to evaluate the state of respiration of the subject, wherein the head tilt mechanism (24) and the jaw thrust mechanism (20) are configured to obtain an opening on the airway, which is achieved by rotating the rotatable handle (30) of the head tilt mechanism (24) and the rotatable handle (38) of the jaw tilt mechanism (20) in response to measurements of the subject's conditions by the sensors
configured to evaluate the state of respiration of the subject.

2. The device (10) of claim 1 wherein the head tilt mechanism (24) comprises:
a locking mechanism;
wherein:
a') the flexible primary strap (12) is configured to be worn on a head (14) of a subject, wherein the flexible primary strap (12) passes over the forehead or over the nose bridge of the subject and the neck of the subject;
b') the rotatable handle (30) is configured such that rotating it in one of a clockwise or counterclockwise direction exerts a force, via a mechanical connection, on the pulley (26) wrapping the end of the flexible primary strap (12) around the pulley (26) effectively applying a force via the flexible primary strap (12) that pulls the head (14) downwards and rotating the rotatable handle (30) in the opposite direction unwraps the flexible primary strap (12) from around the pulley (26) effectively reducing the force that the flexible primary strap (12) exerts on the head (14) allowing the head (14) to move upwards.

3. The device (10) of claim 2, wherein the device (10) comprises a secondary strap (22) that is connected to the primary strap (12) and runs over the top of the head (14) of the subject.

4. The device (10) of claim 2 wherein mechanical connection between the rotatable handle (30) and the pulley (26) is one of:
a) a belt (28); and
b) a gear train.

5. The device (10) of claim 1 wherein the jaw thrust mechanism (20) comprises:
a locking mechanism (54) configured to lock the jaw thrust mechanism (20) to maintain optimal jaw thrust;
wherein:
i) the lower ends of the two drive mechanisms (42) are affixed to the neck support (16) on either side of a head (14) of a subject; and the rotatable handle (38) is configured such that rotating it clockwise or counterclockwise raises or lowers both of the elongated members, thereby pushing the mandible engaging elements (18) upwards or lowering them to move the mandible forwards of backwards.

6. The device (10) of claim 5 wherein the elongated members are one of: screws (44) or racks from rack and pinion gear assemblies.

7. The device (10) of claim 5 wherein the distance (52) between the two drive mechanisms (42) can be adjusted according to the dimensions of the head (14) of the subject.

8. The device (10) of claim 1 wherein the device (10) is configured to be attached to both the head (14) of a subject and to a bed, stretcher, or other flat surface.

9. The device (10) of claim 8 wherein the neck support (16) comprises connectors configured to attach the device (10) to anchorage points on the bed, stretcher, or other flat surface.

10. The device (10) of claim 1 wherein the rotatable handle (30) of the head tilt mechanism (24) is rotated in one of the following ways: manually or by a motor.

11. The device (10) of claim 1 wherein the rotatable handle (38) of the jaw thrust mechanism (20) is rotated in one of the following ways: manually or by a motor.

12. The device (10) of one of claim 10 or claim 11 configured to react to a state of respiratory emergency by automatically opening an airway through rotation of one or both of the head tilt mechanism (24) and the jaw thrust mechanism (20) in response to measurements of the subject's condition by the sensors configured to evaluate the state of respiration of the subject.

## Patentansprüche

1. Eine am Körper tragbare Vorrichtung (10) zum Öffnen und Offenhalten der Atemwege bei einer Person, wobei die Vorrichtung (10) Folgendes umfasst:
a) eine halbstarre Nackenstütze (16), die so ausgebildet ist, dass sie hinter dem Nacken positioniert werden kann und den Nacken der Person stützt;
b) einen Kopfneigungsmechanismus (24), der sich auf einer Seite des Kopfes der Person befindet und an der Nackenstütze befestigt ist, welche einen drehbaren Griff (30) zur Durchführung einer Kopfneigungsbewegung umfasst und durch Veränderung des Kopfwinkels der Person in Rückenlage, um eine Kopfneigungsposition zu erreichen;
c) Unterkiefer-Eingriffselemente (18), die so ausgebildet sind, dass sie der Form der Unterkiefer entsprechen;
d) einen Kieferschubmechanismus (20), welcher an der Nackenstütze befestigt ist, und zwei Antriebsmechanismen (42) umfasst, die durch eine Welle (50) verbunden sind, an deren einem Ende ein drehbarer Griff (38) angebracht ist, sowie aus Zahnrädern, die mit den Antriebsmechanismen (42) in Eingriff stehen, zwei längliche Elemente (44), von denen jeweils eines von jedem Antriebsmechanismus (42) nach oben ragt und so ausgebildet ist, dass es relativ zur Nackenstütze (16) angehoben oder abgesenkt werden kann, wobei die Unterkiefer-Eingriffselemente (18) drehbar am oberen Ende jeweils eines anderen der länglichen Elemente (44) befestigt sind;
e) ein flexibler erster Riemen (12), der mit dem drehbaren Griff (30) verbunden ist und dessen erstes Ende an der Umlenkrolle (26) und dessen zweites Ende an der Nackenstütze (16) befestigt ist, und zwar auf der Seite, die der Seite gegenüberliegt, an der der Kopfneigungsmechanismus (24) an der Nackenstütze (16) befestigt ist, wobei der erste flexible Riemen (12) so ausgebildet ist, dass er sich um die Umlenkrolle wickelt, um den Kopf (14) nach unten zu ziehen, wenn der drehbare Griff (30) in eine erste Richtung gedreht wird, oder um eine Aufwärtsbewegung des Kopfes (14) zu ermöglichen, wenn der drehbare Griff (30) in die entgegengesetzte Richtung gedreht wird;
f) Sensoren, die zur Beurteilung des Atemzustands der Person ausgelegt sind,
wobei der Kopfneigungsmechanismus (24) und der Kieferschubmechanismus (20) so ausgelegt sind, dass sie eine Öffnung der Atemwege bewirken, was durch Drehen des drehbaren Griffs (30) des Kopfneigungsmechanismus (24) und durch Drehen des drehbaren Griffs (38) des Kieferschubmechanismus (20) als Reaktion auf Messungen des Zustands der Person durch die Sensoren erreicht wird.

2. Die Vorrichtung (10) nach Anspruch 1, wobei der Kopfneigungsmechanismus (24) umfasst:
Einen Verriegelungsmechanismus;
wobei:
a') der flexible erste Riemen (12) so ausgebildet ist, dass er am Kopf (14) einer Person getragen werden kann, wobei der flexible erste Riemen (12) über die Stirn oder über den Nasenrücken der Person sowie über den Nacken der Person verläuft;
b') der drehbare Griff (30) so ausgebildet ist, dass durch seine Drehung im Uhrzeigersinn oder gegen den Uhrzeigersinn über eine mechanische Verbindung eine Kraft auf die Umlenkrolle (26) ausgeübt wird, wodurch das Ende der flexible erste Riemen (12) um die Umlenkrolle (26) gewickelt wird wodurch über den flexiblen ersten Riemen (12) effektiv eine Kraft ausgeübt wird, die den Kopf (14) nach unten zieht, und das Drehen des drehbaren Griffs (30) in die entgegengesetzte Richtung der flexible erste Riemen (12) von der Umlenkrolle (26) abwickelt, wodurch die Kraft, die der flexible erste Riemen (12) auf den Kopf (14) ausübt, effektiv verringert wird, sodass sich der Kopf (14) nach oben bewegen kann.

3. Die Vorrichtung (10) nach Anspruch 2, wobei die Vorrichtung ein zweiter Riemen (22) umfasst, der mit dem ersten Riemen (12) verbunden ist und über den Scheitel (14) der Person verläuft.

4. Die Vorrichtung (10) nach Anspruch 2, wobei die mechanische Verbindung zwischen dem drehbaren Griff (30) und der Umlenkrolle (26) eine der folgenden ist:
a) ein Band (28); und
b) ein Zahnradgetriebe.

5. Die Vorrichtung (10) nach Anspruch 1, wobei der Kieferschubmechanismus (20) Folgendes umfasst:
einen Verriegelungsmechanismus (54), der so ausgelegt ist, dass er den Kieferschubmechanismus (20) verriegelt, um einen optimalen Kieferschub aufrechtzuerhalten;
wobei:
i) die unteren Enden der beiden Antriebsmechanismen (42) sind beiderseits des Kopfes (14) einer Person an der Nackenstütze (16) befestigt; und der drehbare Griff (38) ist so ausgebildet, dass durch Drehen im Uhrzeigersinn oder gegen den Uhrzeigersinn beide länglichen Elemente angehoben oder abgesenkt werden, wodurch die Unterkiefer-Eingriffselemente (18) nach oben gedrückt oder abgesenkt werden, um den Unterkiefer nach vorne oder nach hinten zu bewegen.

6. Die Vorrichtung (10) nach Anspruch 5, wobei es sich bei den länglichen Elementen um eines der folgenden handelt: Schrauben (44) oder Zahnstangen aus Zahnstangen- und Ritzel-Getriebebaugruppen.

7. Die Vorrichtung (10) nach Anspruch 5, wobei der Abstand (52) zwischen den beiden Antriebsmechanismen (42) entsprechend den Abmessungen des Kopfes (14) der Person eingestellt werden kann.

8. Die Vorrichtung (10) nach Anspruch 1, wobei die Vorrichtung (10) so ausgelegt ist, dass sie sowohl am Kopf (14) einer Person als auch an einem Bett, einer Trage oder einer anderen ebenen Fläche befestigt werden kann.

9. Die Vorrichtung (10) nach Anspruch 8, wobei die Nackenstütze (16) Befestigungselemente umfasst, die so ausgebildet sind, dass sie die Vorrichtung (10) an Befestigungspunkten am Bett, an der Trage oder an einer anderen ebenen Fläche befestigen.

10. Die Vorrichtung (10) nach Anspruch 1, wobei der drehbare Griff (30) des Kopfneigungsmechanismus (24) auf eine der folgenden Weisen gedreht wird: manuell oder mittels eines Motors.

11. Die Vorrichtung (10) nach Anspruch 1, wobei der drehbare Griff (38) des Kieferschubmechanismus (20) auf eine der folgenden Weisen gedreht wird: manuell oder mittels eines Motors.

12. Die Vorrichtung (10) gemäß Anspruch 10 oder Anspruch 11, die so ausgelegt ist, dass sie auf einen Atemnotzustand reagiert, indem sie durch Drehung des Kopfneigungsmechanismus (24) und/oder des Kieferschubmechanismus (20) automatisch einen Atemweg freigibt, und zwar als Reaktion auf Messungen des Zustands der Person durch die Sensoren, die zur Beurteilung des Atemzustands der Person ausgelegt sind.

## Revendications

1. Dispositif portable (10) pour ouvrir et maintenir des voies respiratoires ouvertes chez un sujet, le dispositif (10) comprenant :
a) un support de cou semi-rigide (16) qui est configuré pour être positionné derrière le cou et est configuré pour supporter le cou du sujet ;
b) un mécanisme d'inclinaison de tête (24) situé sur un côté de la tête du sujet fixé au support de cou (16) comprenant une poignée rotative (30) pour effectuer une manœuvre d'inclinaison de tête en modifiant un angle de tête du sujet lorsqu'il est dans la position allongée afin d'atteindre une position de tête inclinée ;
c) des éléments de mise en prise de mandibule (18) configurés pour correspondre à la forme des mandibules ;
d) un mécanisme de poussée de mâchoire (20) fixé au support de cou (16) comprenant deux mécanismes d'entraînement (42) qui sont reliés par un arbre (50) ayant une poignée rotative (38) fixée à une extrémité de l'arbre (50) et des engrenages qui s'accouplent avec le mécanisme d'entraînement (42), deux éléments allongés (44), un faisant saillie vers le haut à partir du mécanisme d'entraînement (42) et configuré pour être élevé ou abaissé par rapport au support de cou (16), les éléments de mise en prise de mandibule (18) étant fixés de manière pivotante à l'extrémité supérieure d'un élément différent de chacun des éléments allongés (44) ;
e) une bande principale souple (12) reliée à la poignée rotative (30) ayant une première extrémité fixée à la poulie (26) et une seconde extrémité fixée au support de cou (16) à l'opposé du côté au niveau duquel le mécanisme d'inclinaison de tête (24) est fixé au support de cou (16), la bande principale souple (12) étant configurée pour s'envelopper autour de la poulie (26) pour tirer la tête (14) vers le bas lorsque la poignée rotative (30) est tournée dans une première direction ou pour permettre à la tête (14) de se déplacer vers le haut lorsque la poignée rotative (30) est tournée dans une direction opposée ;
f) des capteurs configurés pour évaluer l'état de respiration du sujet, dans lequel le mécanisme d'inclinaison de tête (24) et le mécanisme de poussée de mâchoire (20) sont configurés pour obtenir une ouverture des voies respiratoires, qui est atteinte en faisant tourner la poignée rotative (30) du mécanisme d'inclinaison de tête (24) et la poignée rotative (38) du mécanisme de poussée de mâchoire (20) en réponse à des mesures de l'état du sujet par les capteurs configurés pour évaluer l'état de respiration du sujet.

2. Dispositif (10) selon la revendication 1, dans lequel le mécanisme d'inclinaison de tête (24) comprend :
un mécanisme de verrouillage ;
dans lequel :
a') la bande principale souple (12) est configurée pour être portée sur une tête (14) d'un sujet, dans lequel la bande principale souple (12) passe sur le front ou sur la voûte nasale du sujet et le cou du sujet ;
b') la poignée rotative (30) est configurée de telle sorte que sa rotation dans l'une d'une direction horaire ou antihoraire exerce une force, par l'intermédiaire d'une liaison mécanique, sur la poulie (26) enveloppant l'extrémité de la bande principale souple (12) autour de la poulie (26) appliquant effectivement une force par l'intermédiaire de la bande principale souple (12) qui tire la tête (14) vers le bas et la rotation de la poignée rotative (30) dans la direction opposée libère la bande principale souple (12) d'autour de la poulie (26) réduisant effectivement la force que la bande principale souple (12) exerce sur la tête (14) permettant à la tête (14) de se déplacer vers le haut.

3. Dispositif (10) selon la revendication 2, dans lequel le dispositif (10) comprend une bande secondaire (22) qui est reliée à la bande principale (12) et s'étend sur le sommet de la tête (14) du sujet.

4. Dispositif (10) selon la revendication 2, dans lequel la liaison mécanique entre la poignée rotative (30) et la poulie (26) est l'un de :
a) une courroie (28) ; et
b) un train d'engrenages.

5. Dispositif (10) selon la revendication 1, dans lequel le mécanisme de poussée de mâchoire (20) comprend :
un mécanisme de verrouillage (54) configuré pour verrouiller le mécanisme de poussée de mâchoire (20) pour maintenir une poussée de mâchoire optimale ;
dans lequel :
i) les extrémités inférieures des deux mécanismes d'entraînement (42) sont fixées au support de cou (16) sur un côté ou l'autre d'une tête (14) d'un sujet ; et la poignée rotative (38) est configurée de telle sorte que sa rotation dans la direction horaire ou antihoraire élève ou abaisse les deux éléments allongés, poussant ainsi les éléments de mise en prise de mandibule (18) vers le haut ou les abaissant pour déplacer la mandibule vers l'avant ou vers l'arrière.

6. Dispositif (10) selon la revendication 5, dans lequel les éléments allongés sont l'un de : des vis (44) ou des crémaillères d'ensembles d'engrenages à crémaillère et pignon.

7. Dispositif (10) selon la revendication 5, dans lequel la distance (52) entre les deux mécanismes d'entraînement (42) peut être réglée selon les dimensions de la tête (14) du sujet.

8. Dispositif (10) selon la revendication 1, dans lequel le dispositif (10) est configuré pour être fixé à la tête (14) d'un sujet et à un lit, un brancard ou une autre surface plate.

9. Dispositif (10) selon la revendication 8, dans lequel le support de cou (16) comprend des éléments de liaison configurés pour fixer le dispositif (10) à des points d'ancrage sur le lit, le brancard ou une autre surface plate.

10. Dispositif (10) selon la revendication 1, dans lequel la poignée rotative (30) du mécanisme d'inclinaison de tête (24) est pivotée selon l'une des manières suivantes : manuellement ou par un moteur.

11. Dispositif (10) selon la revendication 8, dans lequel la poignée rotative (38) du mécanisme de poussée de mâchoire (20) est pivotée selon l'une des manières suivantes : manuellement ou par un moteur.

12. Dispositif (10) selon l'une de la revendication 10 ou de la revendication 11, configuré pour réagir à un état d'urgence respiratoire en ouvrant automatiquement des voies respiratoires par l'intermédiaire de la rotation de l'un ou des deux du mécanisme d'inclinaison de tête (24) et du mécanisme de poussée de mâchoire (20) en réponse à des mesures de l'état du sujet par les capteurs configurés pour évaluer l'état de respiration du sujet.
